# EUROPEAN PATENT APPLICATION

(11) **EP 2 278 020 A2**
(43) Date of publication of application: **26.01.2011**
(21) Application number: 10181725.2
(22) Date of filing: 09.04.2003
(51) Int. Cl.: C12N 15/86, C12N 15/861

(54) **Antibody gene transfer and recombinant AAV therefor**

(30) Priority: 09.04.2002 US 371501 P
(62) Divisional of application: 08171295.2
(71) Applicant: Nationwide Children's Hospital, Inc., Columbus, OH 43205 (US)
(72) Inventor: Clark, Kelly Reed, Westerville, OH 43082 (US); Johnson, Philip R., Jr., Wynnewood, PA 19096 (US)
(74) Representative: Donald, Jenny Susan

(57) **Abstract**

The present invention relates generally to the use of recombinant adeno-associated viruses (rAAV) for gene delivery and more specifically to the use of rAAV to deliver antibody genes to target cells in mammals. Administration of rAAV encoding antibodies that neutralize the HIV-1 virus is exemplified.

SEQUENCE LISTING

<110>  Clark et al.

<120>  ANTIBODY GENE TRANSFER AND RECOMBINANT AAV THEREFOR

<130>  28335/39282

<140>  To be assigned
<141>  Herewith

<150>  US 60/371,501
<151>  2002-04-09

<160>  25

<170>  PatentIn version 3.1

<210>  1
<211>  38
<212>  DNA
<213>  Artificial sequence

<220>
<223>  CMV forward primer

<400>  1
tctagaattc tttaattaag tcgttacata acttacgg                              38


<210>  2
<211>  39
<212>  DNA
<213>  Artificial sequence

<220>
<223>  CMV reverse primer

<400>  2
tctagaattc tgcccgggct acaattccgc agcttttag                             39


<210>  3
<211>  36
<212>  DNA
<213>  Artificial sequence

<220>
<223>  BGH forward primer

<400>  3
ttagtgtgcc cgggcactcg ctgatcagcc tcgact                                36


<210>  4
<211>  31
<212>  DNA

<213>  Artificial sequence

<220>
<223>  BGH reverse primer


<400>  4
tagtgtctcg agaatcctcc cccttgctgt c                                    31

<210>  5
<211>  32
<212>  DNA
<213>  Artificial sequence

<220>
<223>  EF1 forward primer

<400>  5
ttagtgtctc gagaactaac atacgctctc ca                                   32


<210>  6
<211>  57
<212>  DNA
<213>  Artificial sequence

<220>
<223>  EF1 Reverse primer

<400>  6
gtgtctgcag gtatttaaat gtgggaattc gtcctaggcc ctcctaccgg tgatctc        57


<210>  7
<211>  35
<212>  DNA
<213>  Artificial sequence

<220>
<223>  Heavy chain cDNA forward primer

<400>  7
tacttcgccc gggctaattc gccgccacca tggaa                                35


<210>  8
<211>  39
<212>  DNA
<213>  Artificial sequence

<220>
<223>  Heavy chain cDNA Reverse primer

<400>  8
tacttcgccc gggctttatt catttacccg gagacaggg                            39

<210>  9
<211>  34
<212>  DNA
<213>  Artificial sequence

<220>
<223>  Light Chain forward primer

<400>  9
cctcacctag gccaccatgg gtgtgccacg ctgg                                    34


<210>  10
<211>  30
<212>  DNA
<213>  Artificial sequence

<220>
<223>  Light chain reverse primer

<400>  10
cctcacctag gattaacact ctcccctgtt                                        30


<210>  11
<211>  20
<212>  DNA
<213>  Artificial sequence

<220>
<223>  CMV forward primer

<400>  11
tggaaatccc cgtgagtcaa                                                   20


<210>  12
<211>  19
<212>  DNA
<213>  Artificial sequence

<220>
<223>  CMV reverse primer

<400>  12
catggtgatg cggttttgg                                                    19


<210>  13
<211>  22
<212>  DNA
<213>  Artificial sequence

<220>
<223>  Synthetic Probe

<400>  13
ccgctatcca cgcccattga tg                                                22

<210>  14
<211>  1431
<212>  DNA
<213>  Homo sapiens

<400>  14
atggaatgga gctgggtctt tctcttcttc ctgtcagtaa ctacaggtgt ccactcccag      60

gttcagctgg ttcagtccgg ggctgaggtg aagaagcctg gggcctcagt gaaggtttct     120

tgtcaggctt ctggatacag attcagtaac tttgttattc attgggtgcg ccaggccccc     180

ggacagaggt ttgagtggat gggatggatc aatccttaca acggaaacaa agaattttca     240

gcgaagttcc aggacagagt cacctttacc gcggacacat ccgcgaacac agcctacatg     300

gagttgagga gcctcagatc tgcagacacg gctgtttatt attgtgcgag agtggggcca     360

tatagttggg atgattctcc ccaggacaat tattatatgg acgtctgggg caaagggacc     420

acggtcatcg tgagctcagc ttccaccaag ggcccatcgg tcttccccct ggcaccctcc     480

tccaagagca cctctggggg cacagcggcc ctgggctgcc tggtcaagga ctacttcccc     540

gaaccggtga cggtgtcgtg gaactcaggc gccctgacca gcggcgtgca caccttcccg     600

gctgtcctac agtcctcagg actctactcc ctcagcagcg tggtgaccgt gccctccagc     660

agcttgggca cccagaccta catctgcaac gtgaatcaca agcccagcaa caccaaggtg     720

gacaagaaag ttgagcccaa atcttgtgac aaaactcaca catgcccacc gtgcccagca     780

cctgaactcc tggggggacc gtcagtcttc ctcttccccc caaaacccaa ggacaccctc     840

atgatctccc ggacccctga ggtcacatgc gtggtggtgg acgtgagcca cgaagaccct     900

gaggtcaagt tcaactggta cgtggacggc gtggaggtgc ataatgccaa gacaaagccg     960

cgggaggagc agtacaacag cacgtaccgg gtggtcagcg tcctcaccgt cctgcaccag    1020

gactggctga atggcaagga gtacaagtgc aaggtctcca acaaagccct cccagccccc    1080

atcgagaaaa ccatctccaa agccaaaggg cagccccgag aaccacaggt gtacaccctg    1140

cccccatccc gggatgagct gaccaagaac caggtcagcc tgacctgcct ggtcaaaggc    1200

ttctatccca gcgacatcgc cgtggagtgg gagagcaatg ggcagccgga gaacaactac    1260

aagaccacgc ctcccgtgct ggactccgac ggctccttct tcctctacag caagctcacc    1320

gtggacaaga gcaggtggca gcaggggaac gtcttctcat gctccgtgat gcatgaggct    1380

ctgcacaacc actacacgca gaagagcctc tccctgtctc cgggtaaatg a             1431

<210>  15
<211>  476
<212>  PRT
<213>  Homo sapiens
<400>  15

Met Glu Trp Ser Trp Val Phe Leu Phe Phe Leu Ser Val Thr Thr Gly
1               5                   10                  15

Val His Ser Gln Val Gln Leu Val Gln Ser Gly Ala Glu Val Lys Lys
            20                  25                  30

Pro Gly Ala Ser Val Lys Val Ser Cys Gln Ala Ser Gly Tyr Arg Phe
        35                  40                  45

Ser Asn Phe Val Ile His Trp Val Arg Gln Ala Pro Gly Gln Arg Phe
    50                  55                  60

Glu Trp Met Gly Trp Ile Asn Pro Tyr Asn Gly Asn Lys Glu Phe Ser
65                  70                  75                  80

Ala Lys Phe Gln Asp Arg Val Thr Phe Thr Ala Asp Thr Ser Ala Asn
                85                  90                  95

Thr Ala Tyr Met Glu Leu Arg Ser Leu Arg Ser Ala Asp Thr Ala Val
            100                 105                 110

Tyr Tyr Cys Ala Arg Val Gly Pro Tyr Ser Trp Asp Asp Ser Pro Gln
        115                 120                 125

Asp Asn Tyr Tyr Met Asp Val Trp Gly Lys Gly Thr Thr Val Ile Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Leu Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220

Gln Thr Tyr Ile Cys Asn Val Asn His Lys Pro Ser Asn Thr Lys Val
225                 230                 235                 240

Asp Lys Lys Val Glu Pro Lys Ser Cys Asp Lys Thr His Thr Cys Pro
                245                 250                 255

Pro Cys Pro Ala Pro Glu Leu Leu Gly Gly Pro Ser Val Phe Leu Phe
            260                 265                 270

Pro Pro Lys Pro Lys Asp Thr Leu Met Ile Ser Arg Thr Pro Glu Val
        275                 280                 285

Thr Cys Val Val Val Asp Val Ser His Glu Asp Pro Glu Val Lys Phe
    290                 295                 300

Asn Trp Tyr Val Asp Gly Val Glu Val His Asn Ala Lys Thr Lys Pro
305                 310                 315                 320

Arg Glu Glu Gln Tyr Asn Ser Thr Tyr Arg Val Val Ser Val Leu Thr
                325                 330                 335

Val Leu His Gln Asp Trp Leu Asn Gly Lys Glu Tyr Lys Cys Lys Val
            340                 345                 350

Ser Asn Lys Ala Leu Pro Ala Pro Ile Glu Lys Thr Ile Ser Lys Ala
        355                 360                 365

Lys Gly Gln Pro Arg Glu Pro Gln Val Tyr Thr Leu Pro Pro Ser Arg
    370                 375                 380

Asp Glu Leu Thr Lys Asn Gln Val Ser Leu Thr Cys Leu Val Lys Gly
385                 390                 395                 400

Phe Tyr Pro Ser Asp Ile Ala Val Glu Trp Glu Ser Asn Gly Gln Pro
                405                 410                 415

Glu Asn Asn Tyr Lys Thr Thr Pro Pro Val Leu Asp Ser Asp Gly Ser
            420                 425                 430

Phe Phe Leu Tyr Ser Lys Leu Thr Val Asp Lys Ser Arg Trp Gln Gln
        435                 440                 445

Gly Asn Val Phe Ser Cys Ser Val Met His Glu Ala Leu His Asn His
    450                 455                 460

Tyr Thr Gln Lys Ser Leu Ser Leu Ser Pro Gly Lys
465                 470                 475

<210>  16
<211>  708
<212>  DNA
<213>  Homo sapiens

<400>  16
atgggtgtgc ccactcaggt cctggggttg ctgctgctgt ggcttacaga tgccagatgt      60

gagatcgttc tcacgcaggc tccaggcacc ctgtctctgt ctccagggga aagagccacc     120

ttctcctgta ggtccagtca cagcattcgc agccgccgcg tacgctggta ccagcacaaa     180

cctggccagg ctccaaggct ggtcatacat ggtgtttcca atagggcctc tggcatctca     240

gacaggttca gcggcagtgg gtctgggaca gacttcactc tcaccatcac cagagtggag     300

cctgaagact ttgcactgta ctactgtcag gtctatggtg cctcctcgta cacttttggc     360

caggggacca aactggagag gaaacgaact gtgcctgcac catctgtctt catcttcccg     420

ccatctgatg agcagttgaa atctgggact gcctctgttg tgtgcctgct gaataacttc     480

tatcccagag aggccaaagt acagtggaag gtggataacg ccctccaatc gggtaactcc     540

caggagagtg tcacagagca ggacagcaag gacagcacct acagcctcag cagcaccctg     600

acgctgagca aagcagacta cgagaaacac aaagtctacg cctgcgaagt cacccatcag     660

ggcctgagat cgcccgtcac aaagagcttc aacaggggag agtgttaa                  708

<210>  17
<211>  235
<212>  PRT
<213>  Homo sapiens

<400>  17

Met Gly Val Pro Thr Gln Val Leu Gly Leu Leu Leu Leu Trp Leu Thr
1               5                   10                  15

Asp Ala Arg Cys Glu Ile Val Leu Thr Gln Ala Pro Gly Thr Leu Ser
            20                  25                  30

Leu Ser Pro Gly Glu Arg Ala Thr Phe Ser Cys Arg Ser Ser His Ser
        35                  40                  45

Ile Arg Ser Arg Arg Val Arg Trp Tyr Gln His Lys Pro Gly Gln Ala
    50                  55                  60

Pro Arg Leu Val Ile His Gly Val Ser Asn Arg Ala Ser Gly Ile Ser
65                  70                  75                  80

Asp Arg Phe Ser Gly Ser Gly Ser Gly Thr Asp Phe Thr Leu Thr Ile
                85                  90                  95

Thr Arg Val Glu Pro Glu Asp Phe Ala Leu Tyr Tyr Cys Gln Val Tyr
            100                 105                 110

Gly Ala Ser Ser Tyr Thr Phe Gly Gln Gly Thr Lys Leu Glu Arg Lys
        115                 120                 125

Arg Thr Val Pro Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Arg Ser
    210                 215                 220

Pro Val Thr Lys Ser Phe Asn Arg Gly Glu Cys
225                 230                 235

<210>  18
<211>  122

<212>  PRT
<213>  Homo sapiens

<400>  18

Leu Leu Glu Ser Gly Pro Gly Leu Leu Lys Pro Ser Glu Thr Leu Ser
1               5                   10                  15

Leu Thr Cys Thr Val Ser Gly Gly Ser Met Ile Asn Tyr Tyr Trp Ser
            20                  25                  30

Trp Ile Arg Gln Pro Pro Gly Glu Arg Pro Gln Trp Leu Gly His Ile
        35                  40                  45

Ile Tyr Gly Gly Thr Thr Lys Tyr Asn Pro Ser Leu Glu Ser Arg Ile
    50                  55                  60

Thr Ile Ser Arg Asp Ile Ser Lys Ser Gln Phe Ser Leu Arg Leu Asn
65                  70                  75                  80

Ser Val Thr Ala Ala Asp Thr Ala Ile Tyr Tyr Cys Ala Arg Val Ala
                85                  90                  95

Ile Gly Val Ser Gly Phe Leu Asn Tyr Tyr Tyr Tyr Met Asp Val Trp
            100                 105                 110

Gly Ser Gly Thr Ala Val Thr Val Ser Ser
        115                 120

<210>  19
<211>  105
<212>  PRT
<213>  Homo sapiens
<400>  19

Leu Thr Gln Ser Pro Ala Thr Leu Ser Leu Ser Pro Gly Glu Arg Ala
1               5                   10                  15

Thr Leu Ser Cys Arg Ala Ser Gln Ser Val Gly Arg Asn Leu Gly Trp
            20                  25                  30

Tyr Gln Gln Lys Pro Gly Gln Ala Pro Arg Leu Leu Ile Tyr Asp Ala
        35                  40                  45

Ser Asn Arg Ala Thr Gly Ile Pro Ala Arg Phe Ser Gly Ser Gly Ser

50                  55                  60

Gly Thr Asp Phe Thr Leu Thr Ile Ser Ser Leu Glu Pro Glu Asp Phe
65                  70                  75                  80

Ala Val Tyr Tyr Cys Gln Gln Arg Ser Asp Trp Pro Arg Thr Phe Gly
                85                  90                  95

Gln Gly Thr Lys Val Glu Ile Lys Arg
            100                 105

<210>  20
<211>  840
<212>  DNA
<213>  Homo sapiens

<220>
<221>  CDS
<222>  (1)..(840)
<223>

<400>  20
atg tgg tgg cgc ctg tgg tgg ctg ctg ctg ctg ctg ctg ctg ctg tgg       48
Met Trp Trp Arg Leu Trp Trp Leu Leu Leu Leu Leu Leu Leu Leu Trp
1               5                   10                  15

ccc atg gtg tgg gcc gat att gtg ctg acg cag tct cca ggc acc ctg       96
Pro Met Val Trp Ala Asp Ile Val Leu Thr Gln Ser Pro Gly Thr Leu
            20                  25                  30

tct ttg tct gca ggg gaa aga gcc acc ctc tcc tgc agg gcc agt cag      144
Ser Leu Ser Ala Gly Glu Arg Ala Thr Leu Ser Cys Arg Ala Ser Gln
        35                  40                  45

agt gtt agc agc ggc tcc tta gcc tgg tac cag cag aaa cct ggt cag      192
Ser Val Ser Ser Gly Ser Leu Ala Trp Tyr Gln Gln Lys Pro Gly Gln
    50                  55                  60

gct ccc agg ctc ctc atc tac ggt gca tcc acc agg gcc act ggc atc      240
Ala Pro Arg Leu Leu Ile Tyr Gly Ala Ser Thr Arg Ala Thr Gly Ile
65                  70                  75                  80

cca gac agg ttc agt ggc agt ggg tct ggg aca gac ttc act ctc aca      288
Pro Asp Arg Phe Ser Gly Ser Gly Ser Gly Thr Asp Phe Thr Leu Thr
                85                  90                  95

atc ggc aga ctg gag cct gaa gat ctc gca gta tat tac tgt cag cag      336
Ile Gly Arg Leu Glu Pro Glu Asp Leu Ala Val Tyr Tyr Cys Gln Gln
            100                 105                 110

tat ggt acc tca ccg tac act ttt ggc cag ggg acc aaa gtg gat atc      384
Tyr Gly Thr Ser Pro Tyr Thr Phe Gly Gln Gly Thr Lys Val Asp Ile

115                 120                 125

aaa cgt ggt ggc ggt ggc tcg ggc ggt ggc ggt tca ggt ggc ggt ggc       432
Lys Arg Gly Gly Gly Gly Ser Gly Gly Gly Gly Ser Gly Gly Gly Gly
    130                 135                 140

tct aga tct tcc cag gtc cag ctt gtg cag tct ggg gct gag gtg aag       480
Ser Arg Ser Ser Gln Val Gln Leu Val Gln Ser Gly Ala Glu Val Lys
145                 150                 155                 160

aag cct ggg tcc tcg gtg cag gtc tcc tgc aag gcc tct gga ggc acc       528
Lys Pro Gly Ser Ser Val Gln Val Ser Cys Lys Ala Ser Gly Gly Thr
                165                 170                 175

ttc agc atg tat ggt ttc aac tgg gtg cga cag gcc cct gga cat ggc       576
Phe Ser Met Tyr Gly Phe Asn Trp Val Arg Gln Ala Pro Gly His Gly
            180                 185                 190

ctt gag tgg atg gga ggg atc atc cct atc ttt ggt aca tca aac tac       624
Leu Glu Trp Met Gly Gly Ile Ile Pro Ile Phe Gly Thr Ser Asn Tyr
        195                 200                 205

gca cag aag ttc cgg ggc aga gtc acg ttt acc gcg gac caa gcc acg       672
Ala Gln Lys Phe Arg Gly Arg Val Thr Phe Thr Ala Asp Gln Ala Thr
    210                 215                 220

agc aca gcc tac atg gag ctg acc aac ctg cga tct gac gac acg gcc       720
Ser Thr Ala Tyr Met Glu Leu Thr Asn Leu Arg Ser Asp Asp Thr Ala
225                 230                 235                 240

gtc tat tat tgt gcg aga gat ttt ggc ccc gac tgg gaa gac ggt gat       768
Val Tyr Tyr Cys Ala Arg Asp Phe Gly Pro Asp Trp Glu Asp Gly Asp
                245                 250                 255

tcc tat gat ggt agt ggc cgg ggg ttc ttt gac ttc tgg ggc cag gga       816
Ser Tyr Asp Gly Ser Gly Arg Gly Phe Phe Asp Phe Trp Gly Gln Gly
            260                 265                 270

acc ctg gtc acc gtc tcc tca tga                                       840
Thr Leu Val Thr Val Ser Ser
        275

<210>  21
<211>  279
<212>  PRT
<213>  Homo sapiens

<400>  21

Met Trp Trp Arg Leu Trp Trp Leu Leu Leu Leu Leu Leu Leu Leu Trp
1               5                   10                  15

Pro Met Val Trp Ala Asp Ile Val Leu Thr Gln Ser Pro Gly Thr Leu
            20                  25                  30

Ser Leu Ser Ala Gly Glu Arg Ala Thr Leu Ser Cys Arg Ala Ser Gln
        35                  40                  45

Ser Val Ser Ser Gly Ser Leu Ala Trp Tyr Gln Gln Lys Pro Gly Gln
    50                  55                  60

Ala Pro Arg Leu Leu Ile Tyr Gly Ala Ser Thr Arg Ala Thr Gly Ile
65                  70                  75                  80

Pro Asp Arg Phe Ser Gly Ser Gly Ser Gly Thr Asp Phe Thr Leu Thr
                85                  90                  95

Ile Gly Arg Leu Glu Pro Glu Asp Leu Ala Val Tyr Tyr Cys Gln Gln
            100                 105                 110

Tyr Gly Thr Ser Pro Tyr Thr Phe Gly Gln Gly Thr Lys Val Asp Ile
        115                 120                 125

Lys Arg Gly Gly Gly Gly Ser Gly Gly Gly Gly Ser Gly Gly Gly Gly
    130                 135                 140

Ser Arg Ser Ser Gln Val Gln Leu Val Gln Ser Gly Ala Glu Val Lys
145                 150                 155                 160

Lys Pro Gly Ser Ser Val Gln Val Ser Cys Lys Ala Ser Gly Gly Thr
                165                 170                 175

Phe Ser Met Tyr Gly Phe Asn Trp Val Arg Gln Ala Pro Gly His Gly
            180                 185                 190

Leu Glu Trp Met Gly Gly Ile Ile Pro Ile Phe Gly Thr Ser Asn Tyr
        195                 200                 205

Ala Gln Lys Phe Arg Gly Arg Val Thr Phe Thr Ala Asp Gln Ala Thr
    210                 215                 220

Ser Thr Ala Tyr Met Glu Leu Thr Asn Leu Arg Ser Asp Asp Thr Ala
225                 230                 235                 240

Val Tyr Tyr Cys Ala Arg Asp Phe Gly Pro Asp Trp Glu Asp Gly Asp
                245                 250                 255

Ser Tyr Asp Gly Ser Gly Arg Gly Phe Phe Asp Phe Trp Gly Gln Gly
            260                 265                 270

Thr Leu Val Thr Val Ser Ser
        275

<210>  22
<211>  1446
<212>  DNA
<213>  Homo sapiens

<220>
<221>  CDS
<222>  (1)..(1446)
<223>

 <400>  22
atg tgg tgg cgc ctg tgg tgg ctg ctg ctg ctg ctg ctg ctg ctg tgg       48
Met Trp Trp Arg Leu Trp Trp Leu Leu Leu Leu Leu Leu Leu Leu Trp
1               5                   10                  15

ccc atg gtg tgg gcc agg atc acg tta aag gaa tcg ggt cct ccg ctg       96
Pro Met Val Trp Ala Arg Ile Thr Leu Lys Glu Ser Gly Pro Pro Leu
            20                  25                  30

gtg aaa ccc aca cag act ctc acg ctg acc tgt tcc ttc tct ggg ttc      144
Val Lys Pro Thr Gln Thr Leu Thr Leu Thr Cys Ser Phe Ser Gly Phe
        35                  40                  45

tca ctg tcc gat ttt gga gtg ggt gta ggc tgg atc cgt cag ccc cca      192
Ser Leu Ser Asp Phe Gly Val Gly Val Gly Trp Ile Arg Gln Pro Pro
    50                  55                  60

gga aag gcc cta gag tgg ctt gca atc att tat tcg gat gat gat aag      240
Gly Lys Ala Leu Glu Trp Leu Ala Ile Ile Tyr Ser Asp Asp Asp Lys
65                  70                  75                  80

cgc tac agc cca tcg ctg aac acc aga ctc acc atc acc aag gac acc      288
Arg Tyr Ser Pro Ser Leu Asn Thr Arg Leu Thr Ile Thr Lys Asp Thr
                85                  90                  95

tcc aaa aat caa gtt gtc ctt gtc atg act agg gtg agt cct gtg gac      336
Ser Lys Asn Gln Val Val Leu Val Met Thr Arg Val Ser Pro Val Asp
            100                 105                 110

aca gcc acg tat ttc tgt gca cac agg agg ggc ccc acc acc ctg ttc      384
Thr Ala Thr Tyr Phe Cys Ala His Arg Arg Gly Pro Thr Thr Leu Phe
        115                 120                 125

ggc gtg ccc atc gcc agg ggc ccc gtg aac gcc atg gac gtg tgg ggc      432
Gly Val Pro Ile Ala Arg Gly Pro Val Asn Ala Met Asp Val Trp Gly
    130                 135                 140

cag ggc atc acc gtg acc atc agc agc gcc agc acc aag ggc ccc agc 480
Gln Gly Ile Thr Val Thr Ile Ser Ser Ala Ser Thr Lys Gly Pro Ser
145                 150                 155                 160

gtg ttc ccc ctg gcc ccc agc agc aag agc acc agc ggc ggc acc gcc 528
Val Phe Pro Leu Ala Pro Ser Ser Lys Ser Thr Ser Gly Gly Thr Ala
                165                 170                 175

gcc ctg ggc tgc ctg gtg aag gac tac ttc ccc gag ccc gtg acc gtg 576
Ala Leu Gly Cys Leu Val Lys Asp Tyr Phe Pro Glu Pro Val Thr Val
            180                 185                 190

agc tgg aac agc ggc gcc ctg acc agc ggc gtg cac acc ttc ccc gcc 624
Ser Trp Asn Ser Gly Ala Leu Thr Ser Gly Val His Thr Phe Pro Ala
        195                 200                 205

gtg ctg cag agc agc ggc ctg tac agc ctg agc agc gtg gtg acc gtg 672
Val Leu Gln Ser Ser Gly Leu Tyr Ser Leu Ser Ser Val Val Thr Val
    210                 215                 220

ccc agc agc agc ctg ggc acc cag acc tac atc tgc aac gtg aac cac 720
Pro Ser Ser Ser Leu Gly Thr Gln Thr Tyr Ile Cys Asn Val Asn His
225                 230                 235                 240

aag ccc agc aac acc aag gtg gac aag aag gtg gag ccc aag agc tgc 768
Lys Pro Ser Asn Thr Lys Val Asp Lys Lys Val Glu Pro Lys Ser Cys
                245                 250                 255

gac aag acc cac acc tgc ccc ccc tgc ccc gcc ccc gag ctg ctg ggc 816
Asp Lys Thr His Thr Cys Pro Pro Cys Pro Ala Pro Glu Leu Leu Gly
            260                 265                 270

ggc ccc agc gtg ttc ctg ttc ccc ccc aag ccc aag gac acc ctg atg 864
Gly Pro Ser Val Phe Leu Phe Pro Pro Lys Pro Lys Asp Thr Leu Met
        275                 280                 285

atc agc agg acc ccc gag gtg acc tgc gtg gtg gtg gac gtg agc cac 912
Ile Ser Arg Thr Pro Glu Val Thr Cys Val Val Val Asp Val Ser His
    290                 295                 300

gag gac ccc gag gtg aag ttc aac tgg tac gtg gac ggc gtg gag gtg 960
Glu Asp Pro Glu Val Lys Phe Asn Trp Tyr Val Asp Gly Val Glu Val
305                 310                 315                 320

cac aac gcc aag acc aag ccc agg gag gag cag tac aac agc acc tac 1008
His Asn Ala Lys Thr Lys Pro Arg Glu Glu Gln Tyr Asn Ser Thr Tyr
                325                 330                 335

agg gtg gtg agc gtg ctg acc gtg ctg cac cag gac tgg ctg aac ggc 1056
Arg Val Val Ser Val Leu Thr Val Leu His Gln Asp Trp Leu Asn Gly
            340                 345                 350

aag gag tac aag tgc aag gtg agc aac aag gcc ttc ccc gcc ccc gag 1104
Lys Glu Tyr Lys Cys Lys Val Ser Asn Lys Ala Phe Pro Ala Pro Glu
        355                 360                 365

aag acc agc aag gcc aag ggc cag ccc agg gag ccc cag gtg tac acc 1152

Lys Thr Ser Lys Ala Lys Gly Gln Pro Arg Glu Pro Gln Val Tyr Thr
    370                 375                 380

ctg ccc ccc agc agg gac gag ctg acc aag aac cag gtg agc ctg acc      1200
Leu Pro Pro Ser Arg Asp Glu Leu Thr Lys Asn Gln Val Ser Leu Thr
385                 390                 395                 400

tgc ctg gtg aag ggc ttc tac ccc agc gac atc gcc gtg gag tgg gag      1248
Cys Leu Val Lys Gly Phe Tyr Pro Ser Asp Ile Ala Val Glu Trp Glu
                405                 410                 415

agc aac ggc cag ccc gag aac aac tac aag acc acc ccc ccc gtg ctg      1296
Ser Asn Gly Gln Pro Glu Asn Asn Tyr Lys Thr Thr Pro Pro Val Leu
            420                 425                 430

gac agc gac ggc agc ttc ttc ctg tac agc aag ctg acc gtg gac aag      1344
Asp Ser Asp Gly Ser Phe Phe Leu Tyr Ser Lys Leu Thr Val Asp Lys
        435                 440                 445

agc agg tgg cag cag ggc aac gtg ttc agc tgc agc gtg aac cac gag      1392
Ser Arg Trp Gln Gln Gly Asn Val Phe Ser Cys Ser Val Asn His Glu
    450                 455                 460

gcc ctg cac aac cac tac acc cag aag agc ctg agc ctg agc ccc ggc      1440
Ala Leu His Asn His Tyr Thr Gln Lys Ser Leu Ser Leu Ser Pro Gly
465                 470                 475                 480

aag tag                                                              1446
Lys

<210>  23
<211>  481
<212>  PRT
<213>  Homo sapiens

<400>  23

Met Trp Trp Arg Leu Trp Trp Leu Leu Leu Leu Leu Leu Leu Leu Trp
1               5                   10                  15

Pro Met Val Trp Ala Arg Ile Thr Leu Lys Glu Ser Gly Pro Pro Leu
            20                  25                  30

Val Lys Pro Thr Gln Thr Leu Thr Leu Thr Cys Ser Phe Ser Gly Phe
        35                  40                  45

Ser Leu Ser Asp Phe Gly Val Gly Val Gly Trp Ile Arg Gln Pro Pro
    50                  55                  60

Gly Lys Ala Leu Glu Trp Leu Ala Ile Ile Tyr Ser Asp Asp Asp Lys
65                  70                  75                  80

Arg Tyr Ser Pro Ser Leu Asn Thr Arg Leu Thr Ile Thr Lys Asp Thr
                85                  90                  95

Ser Lys Asn Gln Val Val Leu Val Met Thr Arg Val Ser Pro Val Asp
            100                 105                 110

Thr Ala Thr Tyr Phe Cys Ala His Arg Arg Gly Pro Thr Thr Leu Phe
        115                 120                 125

Gly Val Pro Ile Ala Arg Gly Pro Val Asn Ala Met Asp Val Trp Gly
    130                 135                 140

Gln Gly Ile Thr Val Thr Ile Ser Ser Ala Ser Thr Lys Gly Pro Ser
145                 150                 155                 160

Val Phe Pro Leu Ala Pro Ser Ser Lys Ser Thr Ser Gly Gly Thr Ala
                165                 170                 175

Ala Leu Gly Cys Leu Val Lys Asp Tyr Phe Pro Glu Pro Val Thr Val
            180                 185                 190

Ser Trp Asn Ser Gly Ala Leu Thr Ser Gly Val His Thr Phe Pro Ala
        195                 200                 205

Val Leu Gln Ser Ser Gly Leu Tyr Ser Leu Ser Ser Val Val Thr Val
    210                 215                 220

Pro Ser Ser Ser Leu Gly Thr Gln Thr Tyr Ile Cys Asn Val Asn His
225                 230                 235                 240

Lys Pro Ser Asn Thr Lys Val Asp Lys Lys Val Glu Pro Lys Ser Cys
                245                 250                 255

Asp Lys Thr His Thr Cys Pro Pro Cys Pro Ala Pro Glu Leu Leu Gly
            260                 265                 270

Gly Pro Ser Val Phe Leu Phe Pro Pro Lys Pro Lys Asp Thr Leu Met
        275                 280                 285

Ile Ser Arg Thr Pro Glu Val Thr Cys Val Val Val Asp Val Ser His
    290                 295                 300

Glu Asp Pro Glu Val Lys Phe Asn Trp Tyr Val Asp Gly Val Glu Val
305                 310                 315                 320

His Asn Ala Lys Thr Lys Pro Arg Glu Glu Gln Tyr Asn Ser Thr Tyr
                325                 330                 335

Arg Val Val Ser Val Leu Thr Val Leu His Gln Asp Trp Leu Asn Gly
            340                 345                 350

Lys Glu Tyr Lys Cys Lys Val Ser Asn Lys Ala Phe Pro Ala Pro Glu
        355                 360                 365

Lys Thr Ser Lys Ala Lys Gly Gln Pro Arg Glu Pro Gln Val Tyr Thr
    370                 375                 380

Leu Pro Pro Ser Arg Asp Glu Leu Thr Lys Asn Gln Val Ser Leu Thr
385                 390                 395                 400

Cys Leu Val Lys Gly Phe Tyr Pro Ser Asp Ile Ala Val Glu Trp Glu
                405                 410                 415

Ser Asn Gly Gln Pro Glu Asn Asn Tyr Lys Thr Thr Pro Pro Val Leu
            420                 425                 430

Asp Ser Asp Gly Ser Phe Phe Leu Tyr Ser Lys Leu Thr Val Asp Lys
        435                 440                 445

Ser Arg Trp Gln Gln Gly Asn Val Phe Ser Cys Ser Val Asn His Glu
    450                 455                 460

Ala Leu His Asn His Tyr Thr Gln Lys Ser Leu Ser Leu Ser Pro Gly
465                 470                 475                 480

Lys

<210>  24
<211>  714
<212>  DNA
<213>  Homo sapiens

<220>
<221>  CDS
<222>  (1)..(714)
<223>

<400> 24
atg cgc ccc acc tgg gcc tgg tgg ctg ttc ctg gtg ctg ctg ctg gcc 48
Met Arg Pro Thr Trp Ala Trp Trp Leu Phe Leu Val Leu Leu Leu Ala
1               5                   10                  15

ctg tgg gcc ccc gcc cgc ggc gcc ctc caa ctg acc cag tct ccg tcc 96
Leu Trp Ala Pro Ala Arg Gly Ala Leu Gln Leu Thr Gln Ser Pro Ser
            20                  25                  30

tcc ttg tct gca tct gtt gga gac aga atc acc atc act tgt cgg gca 144
Ser Leu Ser Ala Ser Val Gly Asp Arg Ile Thr Ile Thr Cys Arg Ala
        35                  40                  45

agt cag ggc gtt acc agt gct tta gcc tgg tat cga cag aag cca gga 192
Ser Gln Gly Val Thr Ser Ala Leu Ala Trp Tyr Arg Gln Lys Pro Gly
    50                  55                  60

agt cct cct caa ctc cta atc tat gat gcc tcc tct tta gaa agt ggg 240
Ser Pro Pro Gln Leu Leu Ile Tyr Asp Ala Ser Ser Leu Glu Ser Gly
65                  70                  75                  80

gtc cca tcg agg ttc agc ggc agt ggt tct ggg acg gag ttc act ctc 288
Val Pro Ser Arg Phe Ser Gly Ser Gly Ser Gly Thr Glu Phe Thr Leu
                85                  90                  95

acc atc agc acc ctg cgg cct gaa gat ttt gca act tat tac tgt caa 336
Thr Ile Ser Thr Leu Arg Pro Glu Asp Phe Ala Thr Tyr Tyr Cys Gln
            100                 105                 110

caa tta cat ttt tac cct cac acc ttc ggc ggc ggc acc agg gtg gac 384
Gln Leu His Phe Tyr Pro His Thr Phe Gly Gly Gly Thr Arg Val Asp
        115                 120                 125

gtg agg cgg act gtg gct gca cca tct gtc ttc atc ttc ccg cca tct 432
Val Arg Arg Thr Val Ala Ala Pro Ser Val Phe Ile Phe Pro Pro Ser
    130                 135                 140

gat gag cag ttg aaa tct ggg act gcc tct gtt gtg tgc ctg ctg aat 480
Asp Glu Gln Leu Lys Ser Gly Thr Ala Ser Val Val Cys Leu Leu Asn
145                 150                 155                 160

aac ttc tat ccc aga gag gcc aaa gta cag tgg aag gtg gat aac gcc 528
Asn Phe Tyr Pro Arg Glu Ala Lys Val Gln Trp Lys Val Asp Asn Ala
                165                 170                 175

ctc caa tcg ggt aac tcc cag gag agt gtc aca gag cag gac agc aag 576
Leu Gln Ser Gly Asn Ser Gln Glu Ser Val Thr Glu Gln Asp Ser Lys
            180                 185                 190

gac agc acc tac agc ctc agc agc acc ctg acg ctg agc aaa gca gac 624
Asp Ser Thr Tyr Ser Leu Ser Ser Thr Leu Thr Leu Ser Lys Ala Asp
        195                 200                 205

tac gag aaa cac aaa gtc tac gcc tgc gaa gtc acc cat cag ggc ctg 672
Tyr Glu Lys His Lys Val Tyr Ala Cys Glu Val Thr His Gln Gly Leu

    210                 215                 220

tcc tcg ccc gtc aca aag agc ttc aac agg gga gag tgt taa             714
Ser Ser Pro Val Thr Lys Ser Phe Asn Arg Gly Glu Cys
225                 230                 235

<210>  25
<211>  237
<212>  PRT
<213>  Homo sapiens

<400>  25

Met Arg Pro Thr Trp Ala Trp Trp Leu Phe Leu Val Leu Leu Leu Ala
1               5                   10                  15

Leu Trp Ala Pro Ala Arg Gly Ala Leu Gln Leu Thr Gln Ser Pro Ser
            20                  25                  30

Ser Leu Ser Ala Ser Val Gly Asp Arg Ile Thr Ile Thr Cys Arg Ala
        35                  40                  45

Ser Gln Gly Val Thr Ser Ala Leu Ala Trp Tyr Arg Gln Lys Pro Gly
    50                  55                  60

Ser Pro Pro Gln Leu Leu Ile Tyr Asp Ala Ser Ser Leu Glu Ser Gly
65                  70                  75                  80

Val Pro Ser Arg Phe Ser Gly Ser Gly Ser Gly Thr Glu Phe Thr Leu
                85                  90                  95

Thr Ile Ser Thr Leu Arg Pro Glu Asp Phe Ala Thr Tyr Tyr Cys Gln
            100                 105                 110

Gln Leu His Phe Tyr Pro His Thr Phe Gly Gly Gly Thr Arg Val Asp
        115                 120                 125

Val Arg Arg Thr Val Ala Ala Pro Ser Val Phe Ile Phe Pro Pro Ser
    130                 135                 140

Asp Glu Gln Leu Lys Ser Gly Thr Ala Ser Val Val Cys Leu Leu Asn
145                 150                 155                 160

Asn Phe Tyr Pro Arg Glu Ala Lys Val Gln Trp Lys Val Asp Asn Ala
                165                 170                 175

Leu Gln Ser Gly Asn Ser Gln Glu Ser Val Thr Glu Gln Asp Ser Lys
          180                 185                 190

Asp Ser Thr Tyr Ser Leu Ser Ser Thr Leu Thr Leu Ser Lys Ala Asp
        195                 200                 205

Tyr Glu Lys His Lys Val Tyr Ala Cys Glu Val Thr His Gln Gly Leu
    210                 215                 220

Ser Ser Pro Val Thr Lys Ser Phe Asn Arg Gly Glu Cys
225                 230                 235

## Description

This application is a continuation in part of U.S. Provisional Application Serial No 60/371,501 filed April 9, 2002.

### Field of Invention

The present invention relates generally to the use of recombinant adeno-associated viruses (rAAV) for gene delivery and more specifically to the use of rAAV to deliver antibody genes to target cells in mammals

### Background

Adeno-associated virus (AAV) is a replication-deficient parvovirus, the single-stranded DNA genome of which is about 4 7 kb in length including 145 nucleotide inverted terminal repeat (ITRs). The nucleotide sequence of the AAV serotype 2 (AAV2) genome is presented in Srivastava et al., J. Virol , 45: 555-564 (1983) as corrected by Ruffing et al., J. Gen. Virol., 75: 3385-3392 (1994). *Cis*-acting sequences directing viral DNA replication (rep), encapsidation/packaging and host cell chromosome integration are contained within the ITRs. Three AAV promoters, p5, p19, and p40 (named for their relative map locations), drive the expression of the two AAV internal open reading frames encoding rep and cap genes. The two rep promoters (p5 and p19), coupled with the differential splicing of the single AAV intron (at nucleotides 2107 and 2227), result in the production of four rep proteins (rep 78, rep 68, rep 52, and rep 40)from the rep gene Rep proteins possess multiple enzymatic properties that are ultimately responsible for replicating the viral genome The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome The life cycle and genetics of AAV are reviewed in Muzyczka, Current Topics in Microbiology and Immunology, 158: 97-129 (1992).

When wild type AAV infects a human cell, the viral genome can integrate into chromosome 19 resulting in latent infection of the cell Production of' infectious virus does not occur unless the cell is infected with a helper virus (for example, adenovirus or herpesvirus) In the case of adenovirus, genes E1A, E1B, E2A, E4 and VA provide helper functions. Upon infection with a helper virus, the AAV provirus is rescued and amplified, and both AAV and adenovirus are produced.

AAV possesses unique features that make it attractive as a vaccine vector for expressing immunogenic peptides/polypeptides and as a vector for delivering foreign DNA to cells, for example, in gene therapy AAV infection of cells in culture is noncytopathic, and natural infection of' humans and other animals is silent and asymptomatic Moreover, AAV infects many mammalian cells allowing the possibility of targeting many different tissues *in vivo.* Moreover, AAV transduces slowly dividing and non-dividing cells, and can persist essentially for the lifetime of those cells as a transcriptionally active nuclear episome (extrachromosomal element) The AAV proviral genome is infectious as cloned DNA in plasmids which makes construction of recombinant genomes feasible. Furthermore, because the signals directing AAV replication, genome encapsidation and integration are contained within the ITRs of the AAV genome, some or all of' the internal approximately 4.3 kb of the genome (encoding replication and structural capsid proteins, rep-cap) may be replaced with foreign DNA such as a gene cassette containing a promoter, a DNA of interest and a polyadenylation signal The rep and cap proteins may be provided *in trans.* Another significant feature of AAV is that it is an extremely stable and hearty virus. It easily withstands the conditions used to inactivate adenovirus (56° to 65°C for several hours), making cold preservation of rAAV-vectors less critical AAV may even be lyophilized Finally, AAV-infected cells are not resistant to superinfection.

HIV-1 is considered to be the causative agent of Acquired Immunodeficiency Syndrome (AIDS) in the United States. As assessed by the World Health Organization, more than 40 million people are currently infected with HIV and 20 million people have already perished from AIDS Thus, HIV infection is considered a worldwide pandemic

There are two currently recognized strains of HIV, HIV-1 and HIV-2. HIV-1 is the principal causes of AIDS around the world. HIV-1 has been classified based on genomic sequence variation into clades. For example, Clade B is the most predominant in North America, Europe, parts of South America and India; Clade C is most predominant in Sub-Saharan Africa; and Clade E is most predominant in southeastern Asia. HIV-1 infection occurs primarily through sexual transmission, transmission from mother to child or exposure to contaminated blood or blood products.

There are two currently recognized strains of HIV, HIV-1 and HIV-2 HIV-1 is the principal causes of AIDS around the world. HIV-1 has been classified based on genomic sequence variation into clades For example, Clade B is the most predominant in North America, Europe, parts of South America and India; Clade C is most predominant in Sub-Saharan Africa; and Clade E is most predominant in southeastern Asia HIV-1 infection occurs primarily through sexual transmission, transmission from mother to child or exposure to contaminated blood or blood products.

HIV-1 consists of a lipid envelope surrounding viral structural proteins and an inner core of enzymes and proteins required for viral replication and a genome of two identical linear RNAs In the lipid envelope, viral glycoprotein 41 (gp 41)anchors another viral envelope glycoprotein 120 (gp 120) that extends from the virus surface and interacts with receptors on the surface of susceptible cells The HIV-1 genome is approximately 10,000 nucleotides in size and comprises nine genes It includes three genes common to all retroviruses, the gag, pol and env genes The gag gene encodes the core structural proteins, the env gene encodes the gp120 and gp41 envelope proteins, and the pol gene encodes the viral enzymes reverse transcriptase (RT), integrase and protease (pro) The genome comprises two other genes essential for viral replication, the tat gene encoding a viral promoter transactivator and the rev gene which also facilitates gene transcription Finally, the nef, vpu, vpr, and vif genes are unique to lentiviruses and encode polypeptides the functions of which are described in Trono, Cell, 82: 189-192 (1995).

The process by which HIV-1 infects human cells involves interaction of proteins on the surface of the virus with proteins on the surface of the cells The common understanding is that the first step in HIV infection is the binding of HIV-1 glycoprotein (gp) 120 to cellular CD4 protein. This interaction causes the viral gp120 to undergo a conformational change and bind to other cell surface proteins, such as CCR5 or CXCR4 proteins, allowing subsequent fusion of the virus with the cell CD4 has thus been described as the primary receptor for HIV-1 while the other cell surface proteins are described as coreceptors for HIV-1

HIV-1 infection is characterized by an asymptomatic period between infection with the virus and the development of AIDS. The rate of progression to AIDS varies among infected individuals. AIDS develops as CD4-positive cells, such as helper T cells and monocytes/macrophages, are infected and depleted. AIDS is manifested as opportunistic infections, increased risk of malignancies and other conditions typical of defects in cell-mediated immunity The Centers for Disease Control and Prevention clinical categories of pediatric, adolescent and adult disease are set out in Table I of Sleasman and Goodenow, J. Allergy Clin. Immunol., 111(2): S582-S592 (2003).

Predicting the likelihood of progression to AIDS involves monitoring viral loads (viral replication) and measuring CD4-positive T cells in infected individuals The higher the viral loads, the more likely a person is to develop AIDS The lower the CD4-positive T cell count, the more likely a person a person is to develop AIDS.

At present, antiretroviral drug therapy (ART) is the only means of treating HIV infection or preventing HIV-1 transmission from one person to another. At best, even with ART, HIV-1 infection is a chronic condition that requires lifelong drug therapy and there can still be a slow progression to disease ART does not eradicate HIV-1 because the virus can persist in latent reservoirs Moreover, treatment regimens can be toxic and multiple drugs must be used daily There thus is an urgent need to develop effective vaccines and treatments for HIV-1 infection.

Over the past several years, progress has been made towards a safe and effective vaccine for HIV infection, and multiple approaches have been taken in animal models and humans. Many of the vaccine candidates have elicited measurable and significant antigen-specific T cell response In contrast, many fail to induce serum antibodies that broadly neutralize primary isolates of HIV-1 Thus, if one considers such antibodies to be an important defense against HIV-1 infection and disease, there remains a significant gap in the design of current HIV-1 vaccine candidates.

There are several hypotheses put forth to explain the lack of neutralizing antibody induction after vaccination with envelope immunogens. First, most anti-envelope antibodies elicited do not recognize the mature oligomeric envelope complex, but rather bind to unprocessed gp160 precursor or monomeric gp120. This is due in part to the trimeric structure of the mature envelope spike, which yields a molecule of low inherent immunogenicity. Extensive glycosylation of surface exposed domains renders a significant portion of the spike non-immunogenic, giving rise to the so-called "silent face" of the molecule Second, the compact structure of the trimeric moiety sterically interferes with antibody recognition of protein epitopes that are located within the core of the trimer Importantly, these same epitopes are readily exposed on the unprocessed gp160 precursor or monomeric gp120 proteins and map to the non-neutralizing face of the protein. Consequently, it has been extremely difficult to isolate human monoclonal antibodies that neutralize primary viral isolates in a broad, cross-clade manner In fact, as few as six such antibodies (b12, 2G12, 2F5, Z13, and 4E10 described in Zwick et al., J. Virol., 75: 12198-12208, 2001 and X5 described in Moulard et al., Proc. Natl. Acad. Sci. USA, 6913-6918, 2002) have been identified despite efforts using a variety of techniques The fact that such antibodies are rare in HIV-1 infected humans serves to underscore the ill-defined but substantial obstacles in eliciting broadly reactive antibodies using traditional methods of vaccination

One potential solution to this problem might be to prophylactically administer antibody preparations (monoclonal or polyclonal) that possess the desired neutralizing activities With regard to HIV-1, studies in non-human primates suggest that passively administered neutralizing antibodies can provide significant protection against SIV/SHIV/HIV infection. This type of "passive immunization" scheme has been successfully applied on a large scale to a targeted population of' infants at risk for serious respiratory syncytial virus infection However, such a strategy for HIV infection has significant drawbacks. It would be cost prohibitive and impractical to frequently administer antibody preparations to large numbers of people for an indefinite period of time

### Summary of Invention

The present invention recognizes the need for development of effective preventative and therapeutic treatments for HIV-1 infection Because of the significant obstacles associated with both active and passive immunization strategies, the present invention utilizes an approach which exploits the existence of the aforementioned neutralizing human monoclonal antibodies against HIV-1 gp160 and the unique gene-delivery properties of AAV

In a first aspect, the invention provides rAAV genomes. The rAAV genomes comprise AAV ITRs flanking a gene cassette of DNA encoding one or more antibody polypeptides operatively linked to transcriptional control DNA, specifically promoter DNA and polyadenylation signal sequence DNA, functional in target cells The gene cassette may also include intron sequences to facilitate processing of the RNA transcript when expressed in mammalian cells The rAAV genomes of the invention lack AAV rep and cap DNA AAV DNA in the rAAV genomes may be from any AAV serotype for which a recombinant virus can be derived.

In particular, the invention contemplates a dual promoter gene cassette which encodes light and heavy chain polypeptides In one embodiment, the gene cassette contains the following: (1) two constitutive promoters that are active in the cell that will be transduced, (2) several unique restriction enzyme sites to allow for the rapid replacement of promoto elements or heavy and light chain coding sequences, (3) unique restriction sites that facilitate in-frame antibody gene cloning, (4) a strong transcriptional termination site 3' to the first expression cassette to reduce possible promoter interference and (5) polynucleotide sequences encoding both the heavy and light chain of a monoclonal antibody of interest each inserted under the transcriptional control of ne of the two promoters.

The invention contemplates rAAV genomes which express antibodies directed to viral proteins (for example, proteins of HIV, Hepatitis B virus, Hepatitis C virus, Epstein Barr Virus and Respiratory Syncytial Virus) and bacterial proteins as well as other proteins associated with chronic disease states (such as a protein expressed only when the disease state occurs or a protein whose expression is upregulated compared to expression in the absence of the disease state) where provision of antibodies would be an effective treatment Examples of chronic disease states include cancers, inflammatory diseases such as rheumatoid arthritis and inflammatory bowel diseases, and prion associated diseases such as Mad Cow Disease, Creutzfeldt-Jakob disease, Gerstmann-Straussler-Scheinker syndrome, fatal familial insomnia, kuru, and Alpers syndrome Genomes encoding monoclonal antibodies which neutralize primary HIV-1 isolates such as monoclonal antibodies IgG1b12, 2F5, Z13, 45E10, , F105 and X5 are specifically contemplated These antibodies are variously described in Zwick et al., J. Virol., 75: 12198-12208, 2001; Baba et al., Nat. Med. 6: 200-206, 2000; and Moulard et al., Proc. Natl. Acad. Sci. USA, 6913-6918, 2002) Amino acid sequences of Z13 are set out in Genebank Accession Nos. AY035845 and AY035846 (SEQ ID NOs: 18 and 19) and heavy and light chain DNA and amino acid sequences of 2F5 are respectively in SEQ ID NOS: 22 AND 23 and SEQ ID NOS: 24 and 25 Specifically exemplified herein is a dual promoter gene cassette comprising the human CMV immediate early promoter/enhancer, the SV40 small T-antigen intron, b12 heavy chain coding sequences, the bovine growth hormone polyadenylation site, the human elongation factor-1α promoter modified using the R segment and part of the U5 sequence of the HTLV Type 1 Long Terminal Repeat, the I117 intron, b12 light chain coding sequences and the SV40 polyadenylation site. A rAAV genome comprising that gene cassette is designated the rAAV/IgG1b12 genome. Also specifically exemplified herein is a gene cassette comprising the human CMV immediate early promoter/enhancer, the SV40 small T-antigen intron, X5 heavy chain variable region coding sequences, a (Gly₃Ser)₄ linker, X5 light chain variable region coding sequences and the SV40 polyadenylation site A rAAV genome comprising that gene cassette is designated the rAAV/ScFvX5 genome.

The invention contemplates antibody polypeptides encoded by the rAAV genomes may be intact immunoglobulin molecules of' any class (IgG, IgA, IgD, IgM or IgE) or subclasses thereof, tetramers, dimers, single chain antibodies, bifunctional antibodies, chimeric antibodies, humanized antibodies, wholly novel recombinant antibodies, antibodies synthesized *de novo* by chemical or biological means, Fab fragments, F(ab)₂', and other immunoactive portions, fragments, segments and other smaller or larger partial antibody structures, wherein all possess sufficient binding activity so as to be therapeutically useful within the methods of the present invention. In addition, since the invention contemplates the use of "humanized" antibodies, for example, some variation in the identity of the framework and/or complementarity determining regions (*i.e*., the CDRs or hypervariable regions of the heavy and light chain variable regions of said antibodies that are critical to determining the antigenic specificity of the antibodies) is both permitted and expected. Thus, in accordance with the present invention, the amino acid sequence(s) of the antibody polypeptides useful in the present invention may show some sequence variation from those antibodies of known utility in treating infections (acute and chronic infections) and chronic diseases Such sequence variations may be as much as 5%, thereby an antibody sequence useful herein has at least a 95% identity with an antibody of known utility. As noted above, antibody polypeptides may be (or be derived from) any antibody class. Antibody class may be selected (or modified) by those skilled in the art taking into account the infection and/or disease state being treated and the desired action(s) and site(s) of action of'the antibody polypeptides.

In another aspect, the invention provides DNA vectors comprising rAAV genomes of the invention The vectors are transferred to cells permissible for infection with a helper virus of AAV (e.g., adenovirus, E1-deleted adenovirus or herpesvirus) for assembly of'the rAAV genome into infectious viral particles. Techniques to produce rAAV particles, in which a AAV genome to be packaged, rep and cap genes, and helper virus functions are provided to a cell are standard in the art. Production of rAAV requires that the following components are present within a single cell (denoted herein as a packaging cell): a rAAV genome, AAV rep and cap genes separate from the rAAV genome, and helper virus functions. The AAV rep and cap genes may be from any AAV serotype for which recombinant virus can be derived and may be from a different AAV serotype than the rAAV genome ITRs

A method of generating a packaging cell is to create a cell line that stably expresses all the necessary components for AAV particle production. For example, a plasmid (or multiple plasmids) comprising a rAAV genome , AAV rep and cap genes separate from the rAAV genome, and a selectable marker, such as a neomycin resistance gene, are integrated into the genome of a cell. The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells

The invention thus provides packaging cells that produce infectious rAAV. In one embodiment packaging cells may be stably transformed cancer cells such as HeLa cells, 293 cells and PerC.6 cells (a cognate 293 line) In another embodiment, packaging cells are cells that are not transformed cancer cells such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells)

In another aspect, the invention provides rAAV (*i.e*., infectious encapsidated rAAV particles) comprising a rAAV genome of the invention In one embodiment, the rAAV is rAAV/IgG1b12. In another embodiment the rAAV is rAAV/ScFvX5. The rAAV may be purified by methods standard in the art such as by column chromatography or cesium chloride gradients.

In another embodiment, the invention contemplates compositions comprising rAAV of the present invention. These compositions may be used to treat and/or prevent viral infections (acute and chronic viral infections) in particular AIDS, bacterial infections (acute, subacute and chronic bacterial infections) and other chronic disease states. In one embodiment, compositions of the invention comprise a rAAV encoding an antibody polypeptide of interest In other embodiments, compositions of the present invention may include two or more rAAV encoding different antibody polypeptides of interest In particular for neutralizing HIV-1, administration of a rAAV mixture which results in expression and secretion of several anti-HIV-1 antibody polypeptides may increase neutralization of the virus. Administration may precede, accompany or follow ARI.

Compositions of the invention comprise rAAV in a pharmaceutically acceptable carrier. The compositions may also comprise other ingredients such as diluents and adjuvants. Acceptable carriers, diluents and adjuvants are nontoxic to recipient and are preferably inert at the dosages and concentrations employed, and include buffers such as phosphate, citrate, or other organic acids; antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics or polyethylene glycol (PEG).

Titers of rAAV to be administered in methods of the invention will vary depending, for example, on the particular rAAV, the mode of administration, the treatment goal, the individual, and the cell type(s) being targeted, and may be determined by methods standard in the art.

Methods of transducing a target cell with rAAV, *in vivo or in vitro,* are contemplated by the invention. The *in vivo* methods comprise the step of administering an effective dose or doses of a composition comprising a rAAV of the invention to an animal (including a human being) in need thereof. If the dose is administered prior to infection by a virus or development of a chronic disease state, the administration is prophylatic. If the dose is administered after infection by a virus or development of a chronic disease state, the administration is therapeutic. An effective dose is a dose sufficient to alleviate (eliminate or reduce) at least one symptom associated with the infection or disease state being treated. In one embodiment, alleviation of symptoms prevents progression of a viral infection to a disease state. In another embodiment, alleviation of symptoms prevents progression to, or progression of, a disease state (whether or not caused by a viral infection). Viral infections (including acute and chronic viral infections), bacterial infections (including acute, subacute and chronic infections) and chronic diseases states of a patient to be treated include, but are not limited to, HIV-1 infection, Hepatitis B virus infection, Hepatitis C virus infection, Epstein Barr Virus infection, Respiratory Syncytial Virus infection, osteomyelitis, tuberculosis, rheumatoid arthritis, inflammatory bowel disease, transmissible spongiform encephalopathies such as Creutzfeldt-Jakob disease and kuru, Gerstmann-Straussler-Scheinker syndrome, fatal familial insomnia, Alpers syndrome, and cancers. In the methods, rAAV encoding antibody polypeptides specifically reactive with a proteins or peptide associated with the infection or disease state are administered Administration of' an effective dose of'the compositions may be by routes standard in the art, for example, parenteral, intravenous, oral, buccal, nasal, pulmonary, intracranial, intraosseous, intraocular, rectal, or vaginal. Route(s) of administration and seiotype(s) of AAV components of rAAV (in particular, the AAV ITRs and capsid protein) of the invention may be chosen and/or matched by those skilled in the art taking into account the infection and/or disease state being treated and the target cells/tissue(s) that are to express the antibody polypeptides.

In particular, actual adminstration of rAAV of the present invention may be accomplished by using any physical method that will transport the rAAV recombinant vector into the target tissue of an animal. Simply resuspending a rAAV in phosphate buffered saline has been demonstrated to be sufficient to provide a vehicle useful for muscle tissue expression, and there are no known restrictions on the carriers or other components that can be coadministered with the vector (although compositions that degrade DNA should be avoided in the normal manner with vectors). Capsid proteins of a rAAV may be modified so that the rAAV is targeted to a particular target tissue of interest such as muscle Pharmaceutical compositions can be prepared as injectable formulations or as topical formulations to be delivered to the muscles by transdermal transport Numerous formulations for both intramuscular injection and transdermal transport have been previously developed and can be used in the practice of the invention. The rAAV can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of rAAV as a free acid (DNA contains acidic phosphate groups) or a pharmacologically acceptable salt can be prepared in water suitably mixed with a surfactant such as hydroxpropylcellulose A dispersion of rAAV can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating actions of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of'surfactants The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonger absorption of the injectable compositions can be brought about by use of agents delaying absorption, for example, aluminum monostearate and gelatin.

Sterile injectable solutions are prepared by incorporating rAAV in the required amount in the appropriate solvent with various of the other ingredients enumerated above, as required, followed by filter sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from the previously sterile-filtered solution thereof.

Transduction with rAAV can also be carried out *in vitro.* In one embodiment, desired target muscle cells are removed from the subject, transduced with rAAV and reintroduced into the subject Alternatively, syngeneic or xenogeneic muscle cells can be used where those cells will not generate an inappropriate immune response in the subject.

Suitable methods for the transduction and reintroduction of transduced cells into a subject are known in the art. In one embodiment, cells can be transduced *in-vitro* by combining rAAV with muscle cells, *e.g*., in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using *e g*., a catheter.

Transduction of cells with rAAV of the invention results in sustained expression of' antibody polypeptide(s). The present invention thus provides methods of delivering rAAV which express antibody polypeptides to an animal, prferably a human being. These methods include transducing tissues (including but not limited to muscle, liver and brain) with one or more rAAV of the present invention Transduction may be carried out with gene cassettes comprising tissue specific control elements For example, one embodiment of the invention provides methods of transducing muscle cells and muscle tissues directed by muscle specific control elements, including, but not limited to, those derived from the actin and myosin gene families, such as from the myoD gene family (See: Weintraub et al., Science 251: 761-766, 1991), the myocyte-specific enhancer binding factor MEF-2 (Cserjesi and Olson, Mol. Cell Biol. 11: 4854-4862, 1991), control elements derived from the human skeletal actin gene (Muscat et al., Mol Cell Biol. 7: 4089-4099, 1987), the cardiac actin gene, muscle creatine kinase sequence elements (See: Johnson et al. Mol.. Cell Biol. 9:3393-3399, 1989) and the murine creatine kinase enhancer (mCK) element, control elements derived from the skeletal fast-twitch troponin C gene, the slow-twitch cardiac troponin C gene and the slow-twitch troponin I gene: hypozia-inducible nuclear factors (Semenza et al., Proc. Natl. Acad Sci USA 88: 5680-5684, 1991), steroid-inducible elements and promoters including the glucocorticoid response element (GRE) (See: Mader and White, Proc Natl. Acad Sci. USA 90: 5603-5607, 1993), and other control elements.

Muscle tissue is a attractive target for *in vivo* gene delivery and gene therapy, because it is not a vital organ and is easy to access. To carry out the delivery of the rAAV in the methods of'the present invention to muscle cells and tissue, the use of single-chain antibody (scFv) or Fab derivatives is contemplated to facilitate more efficient antibody secretion from the muscle tissue. One single chain antibody contemplated by the invention is the HIV-1 neutralizing single chain antibody X5, the DNA and amino acid sequences of which are set out in SEQ ID NOs: 20 and 21, respectively In addition, rAAV based on alternate serotypes (e g AAV-1 and AAV-5) may transduce skeletal myocytes more efficiently than AAV-2

rAAV have been shown to transduce muscle cells or muscle tissue with high efficiency and direct the long-term expression of a variety of transgenes Because of the flexibility of this system, it is contemplated that light and heavy chain antibody genes can be incorporated into a single rAAV, and the antibody-expressing rAAV can then be used to transduce muscle *in vivo.* The invention contemplates sustained expression of biologically active antibody polypeptides from transduced myofibers

By "muscle cell" or "muscle tissue" is meant a cell or group of cells derived from muscle of' any kind, including skeletal muscle, smooth muscle, *e.g*. from the digestive tract, urinary bladder and blood vessels, cardiac, and excised from any area of the body Such muscle cells may be differentiated or undifferentiated, such as myoblasts, myocytes, myotubes, cardiomyocytes and cardiomyoblast. Since muscle tissue is readily accessible to the circulatory system, a protein produced and secreted by muscle cells and tissue *in vivo* will logically enter the bloodstream for systemic delivery, thereby providing sustained, therapeutic levels of protein secretion from muscle.

The term "transduction" is used to refer to the delivery of antibody polypeptide DNA to a recipient cell either *in vivo* or *in vitro,* via a replication-defificient rAAV of the invention resulting in expression of a functional antibody polypeptide by the recipient cell

It is known that antibodies directed to HIV-1 can neutralize the virus by prohibiting viral binding to its receptor(s) The invention provides methods of administering an effective dose (or doses) of rAAV of the present invention that encode antibody polypeptide(s) that neutralize a virus to a patient in need thereof. Neutralization according to the invention is a reduction in infectivity of a primary viral isolate as measured by an *in vitro or in vivo* assay known in the art. Multiple assays are known in the art. Neutralization may involve one or more of the following: binding of antibody to antigen on the surface of the virus blocking interaction of the virus with a receptor on a cell, binding of' antibody to antigen on the surface of the virus resulting in complement-mediated lysis and/or phagocytosis of the virus, binding of antibody to antigen on the surface of' cells infected with the virus resulting in activation of Fc-mediated effector systems and lysis/clearance of the infected cell by antibody-dependent cellular cytotoxicity (ADCC) or complement-dependent cytotoxicity, binding of antibody to antigen on the surface of cells infected with the virus resulting in inhibition of vital replication, binding of' antibody to antigen on the surface of cells infected with the virus resulting in inhibition of virus release from the infected cells, and binding of antibody to antigen on the surface of cells resulting in inhibition of cell-cell transmission of the virus. Neutralization by intracellular immunization is also contemplated. Furthermore, neutralization of bacteria is contemplated

Neutralization may result in clearance of a virus or bacteria from the patient (i.e., sterilization) or may slow progression to a disease state caused by a virus or bacteria. In one embodiment, methods of the invention include the administration of an effective dose (or doses) of rAAV of the invention encoding HIV-1 neutralizing antibody polypeptides to prevent progression of a patient infected with HIV-1 to AIDS Preferred methods result in one or more of the following in the individual : a reduction of viral loads, maintenance of low viral loads, an increase in CD4-positive T cells, stabilization of CD4-positive T cells, reduced incidence or severity of opportunistic infections, reduced incidence of malignancies, and reduced incidence or severity of conditions typical of defects in cell-mediated immunity. The foregoing are each in comparison to an individual that, according to the art, has progressed or will likely progress to AIDS.

As described herein (see Example 5), significant levels of HIV-1 neutralizing activity are found in the sera of mice for over six months after a single intramuscular administration of a rAAV vector of the present invention which expresses the anti-HIV-1 monoclonal antibody IgG1b12 This approach allows for predetermination of antibody affinity and specificity prior to "immunization", and avoids the need for an active humoral immune response against the HIV envelope protein.

### Brief Description of Drawing

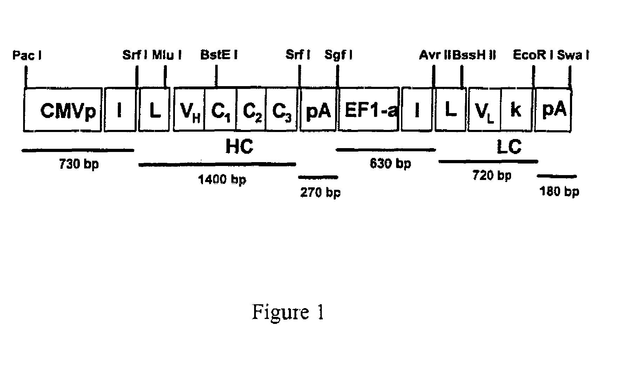

Figure 1 depicts a dual promoter rAAV genome designated pCMV/HC/EF1a/LC. Unique restriction sites are labeled at the top of the schematic. Genome components are labeled as follows: "HC" and "LC" denote the heavy and light chain antibody genes, respectively, "CMVp" represents the human CMV immediate early promoter/enhancer, and "I" denotes the SV40 small T-antigen intron. Antibody leader sequences are labeled "L", and "pA" denotes the bovine growth hormone polyadenylation site. The second transcriptional unit contains the human elongation factor -1α (EF-1α) promoter, and has been modified to enhance stability of DNA and RNA using the R segment and part of'the U5 sequence (R-U5') of the HTLV Type 1 Long Terminal Repeat. This promoter also contains the I117 intron, which is derived from plasmid pGT62LacZ (InVivoGen Inc.). The light chain polyadenylation site is from SV40.

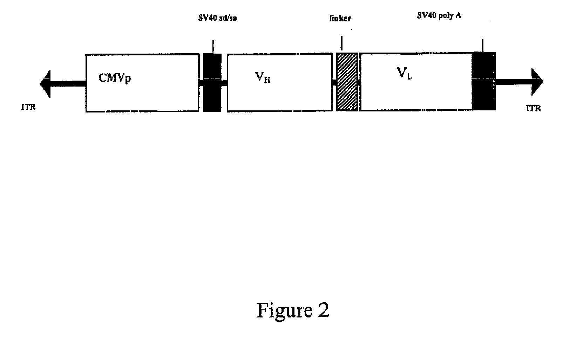

Figure 2 depicts the rAAV/X5 genome that contains a gene cassette comprising the human CMV immediate early promoter/enhancer (denoted "CMV"), the SV40 small T-antigen intron ("SV40 sd/sa"), X5 heavy chain variable region coding sequences ("V_{H}"), a (Gly₃Ser)₄ linker ("linker"), X5 light chain variable region coding sequences ("V_{L}") and the SV40 polyadenylation site ("SV40 poly A").

### Detailed Description

The following examples illustrate the invention wherein Example 1 describes construction of' a dual promoter rAAV for antibody polypeptide expression, Example 2 describes rAAV production, Example 3 describes production of circulating IgG₁ in rAAV transduced mice, Example 4 describes neutralization of HIV-1 activity by muscle-derived IgG1b12 and Example 5 describes rAAV persistence and IgG1b12 production in muscle. Example 6 describes construction of a single promoter rAAV for antibody polypeptide expression Example 7 describes rAAV administration to macaques and Example 8 to humans. Example 9 describes rAAV useful in the invention including rAAV that encode antibody polypeptides other than those useful for viral infections

### Example 1

### Construction of a dual promoter rAAV for antibody expression.

To achieve efficient antibody expression within target muscle cells, a dual promoter rAAV was constructed that resulted in optimal co-expression of heavy and light chain proteins within the same transduced cell. As shown in Figure 1, the resulting dual promoter rAVV had the following features: (1) two constitutive promoters that are active in skeletal muscle in the context of a rAAV vector (hCMV promoter/enhancer and the human EF1-alpha promoter); (2) several unique 8 basepair restriction enzyme sites incorporated into the vector to allow for the rapid replacement of promotor elements or heavy and light chain coding sequences; (3) site-directed mutagenesis was performed on the heavy and light chain leader peptide sequences of IgG1b12 to introduce unique restriction sites (Mlu I for the heavy chain leader and BssH II for the light chain leader) that facilitate in-frame antibody gene cloning; (4) the IgG1b12 heavy chain introns were removed by RT-PCR to reduce vector size and remain within the packaging limit of wild-type AAV; and (5) a strong transcriptional termination site 3' to the first expression cassette to reduce possible promoter interference. Lastly, to enable high-titer rAAV/IgG1b12 vector production using a stable producer cell line approach (Clark et al., Hum. Gene Ther. 6:1329-1341, 1995), the rAAV/IgG1b12 plasmid vector sequences were cloned into a larger tripartite plasmid (pAAV/IgG1b12/rep-cap/neotk) that also contains the AAV-2 rep-cap helper sequences and a neomycin resistance gene as previously described (Clark et al., Hum. Gene Ther. 6:1329-1341, 1995). The tripartite plasmid was then used to generate an optimal HeLa based, rAAV/IgG1b12 producer cell line (CE71).

The coding sequences for the light and heavy chains of'the human monoclonal antibody, IgG1b12, were derived from plasmid pDR12 and are set out at SEQ ID NOS: 14 and 16, respectively herein. The resulting amino acid sequences for the heavy and light chains of IgG1b12 are set out as SEQ ID NOS: 15 and 17, respectively herein Isolation of IgG1b12 and plasmid pDR12 have been described in Burton et al., Proc Natl Acad. Sci. USA 88: 10134-10137,1991 and Science 266: 1024-1027 (1994).

The dual promoter rAAV cloning plasmid pAAV/IgG1b12 was constructed sequentially as follows. First, plasmid pCMV/β (Clontech) was digested with Pst I and the 2.7 kb vector plasmid DNA fragment isolated and re-ligated with itself to generate a ampicillin resistant vector (pB) PCR was then used to amplify the hCMV promoter/enhancer and SV40 intron (808 bp) from plasmid pCMV/β and was carried out with the following primers: CMV forward: TCIAGAATTCTTTAATTAAGTCGTTA CATAACTTACGG (SEQ ID NO: 1); CMV reverse: TCTAGAATTCTGCCCGGG CTACAATTCCGCAGCTTTTAG (SEQ ID NO: 2).

The resulting fragment was cloned into the unique EcoR I site of plasmid pB to generate plasmid pCMV. These primers were designed with EcoR I sites flanking unique Pac I and SrfI sites at the 5' and 3' ends. Subsequently, PCR was used to amplify the bovine growth hormone polyadenylation signal (190 bp) and the EF1-α promoter (770 bp) separately using plasmid pGT621acZ as a template (InVivoGen) and the following primers respectively: BGH forward: TTAGTGTGCCCGGGCACTCGCTGATCAGCCTCGACT (SEQ ID NO: 3); BGH reverse: TAGTGTCTCGAGAATCCTCCCCCTTGCTGTC (SEQ ID NO: 4); EF1 forward: TTAGTGTCICGAGA ACTAACATACGCTCTCCA (SEQ ID NO: 5); EF1 reverse: GTGTCTGCAGGTATTTAAATGTGGGAATTCGTCCTAGGCCCIC CTACCGGTGATCTC (SEQ ID NO: 6) The resulting PCR fragments were subsequently Xho I digested, re-ligated, and a second round of PCR performed with the BGH forward and EF1-α reverse primers (SEQ ID NOS: 3 and 6) to generate a single DNA fragment. These primers incorporated a 5' Srf I site and 3' Avr II, EcoR I, Swa I, and Pst I sites, respectively. This 960 bp DNA fragment was directionally cloned into plasmid pCMV at the unique Srf I and Pst I sites. The resulting plasmid (pCMV/EF1) now possessed the CMV promoter with a BGH polyadenyation site followed by the EF1-alpha promoter.

A 270 base pair DNA fragment containing the SV40 polyadenylation signal (isolated from plasmid pGT621acZ) was directionally cloned into the EcoR I/Swa I sites of plasmid pCMV/EF1 to yield pCMV/EF1a The IgG1b12 heavy chain cDNA (1,463 bp) was isolated by transfecting CHO cells with plasmid pDR12 and isolating total RNA. The RNA was subjected to RT-PCR and cloned into the pZero vector (Invitrogen) with the following primers: heavy chain cDNA forward: TACTTCGCCCGGGCTAATTCGCCGCC ACCATGGAA (SEQ ID NO: 8); heavy chain cDNA reverse: TACTT CGCC CGGGCTTTATTCATTTACCCGGAGACAGGG (SEQ ID NO: 9). These primers incorporated flanking Srf I sites that facilitated the cloning of the IgG1b12 heavy chain into the unique SrfI site in plasmid pCMV/EF1a, yielding plasmid pCMV/HC/EF1a The IgG1b12κ light chain gene was PCR amplified directly from plasmid pDR12 and the 720 bp product cloned into plasmid pZero with the following primers: light chain forward: CCTCACCTAGGCCACCA TGGGTGTGCCACGCTGG (SEQ ID NO: 9); light chain reverse: CCTCACCTAGGATTAACACTCTCCCCTGTT (SEQ ID NO: 10). The light chain primers incorporated flanking Avr II restriction sites that were used to clone the kappa light chain into the Avr II site of plasmid pCMV/HC/EF1a to generate plasmid pCMV/HC/EF1a/LC.

Site directed mutagenesis was then used to introduce a unique Mlu I restriction site into the heavy chain leader peptide sequence and a similar strategy was employed to introduce a BssH I site into the light chain leader peptide sequence. The dual expression cassette was then isolated as a 4.5 kb Pac I/Srf I DNA fragment and cloned between the AAV ITRs of plasmid pAAV/β-gal/rep-cap/neotk (Clark et al., Hum. Gene Therapy 6: 1329-1341, 1995) to generate pAAV/IgG1b12/rep-cap/neotk. This tripartite plasmid contains the native rep-cap AAV helper sequences, as well as, the neomycin resistance gene for stable cell line selection.

The ability of'the plasmid pAAV/IgG1b12/rep-cap/neotk and rAAV/IgG1b12 to produce human IgG₁ antibody was initially confirmed *in vitro* using several transformed cell lines (CHO-K1, HeLa, COS-7 and C2C12) Following plasmid transfection or rAAV/IgG1b12 transduction using standard methods in the art, cell culture supernatant was analyzed using the 1 human IgG subtype 1 ELISA Immunoassay kit (The Binding Site) as directed by the manufacturer. The sensitivity of this assay is 2 9 mg/ml of human IgG. This assay determined that the cell culture supernatent contained detectable levels of human IgG₁.

### Example 2

### rAAV production

rAAV/IgG1b12 was produced and purified using methods known in the art (Clark et al., Hum. Gene Therapy 10: 1031-1039, 1999; Clark et al., Hum. Gene Therapy 6: 1329-1341, 1995) Briefly, a producer cell line (CE71) was isolated following HeLa cell transfection with plasmid pAAV/IgG1b12/rep-cap/neotk and subsequent G418 (700 µg/ml) drug selection Two hundred individual cell lines were screened following wild-type adenovirus type 5 infection (moi = 20) and CE71 was identified as producing the highest DNase resistant particles (DRP) per cell (10⁴ DRP/cell). For large scale vector production, 10¹⁰ CE71 cells were expanded in a Corning Cell Cube adherent cell bioreactor and subsequently infected with wild-type Ad 5 (moi = 20). Following development of adenovirus CPE (72 hr), rAAV/IgG1b12 was purified from the crude CE71 cell lysate using heparin chromatograph as previously detailed (Clark et al., Hum. Gene Therapy 10: 1031-1039, 1999) DRP titers were determined for purified rAAV/IgG1b12 by real time PCR methodology utilizing a Prism 7700 Taqman sequence detector system (PE Applied Biosystems) as detailed in Clark *et al.,* 1999. The primer and fluorescent probe set used for rAAV/IgG1b12 quantitation were as follows; CMV forward primer: 5'-TGGAAATCCCCGTGAGTCAA-3' (SEQ ID NO: 11), CMV reverse primer: 5'-CATGGTGATGCGGTTTTGG-3 (SEQ ID NO: 12)', and probe, 5-FAM-CCGCTATCCACGCCCATIGATG-TAMRA-3' (SEQ ID NO: 13)

An infectious rAAV/IgG1b12 titer was determined using serial dilutions of the rAAV/IgG1b12 stock and infecting a rep-cap expressing cell line (C12) in the presence of adenovirus. An end point titer determination was made based on quantitative PCR detection of replicating rAAV/IgG1b12 genomes in C12 cells, as previously described (Clark et al., Gene Therapy 3: 1124-1132, 1996) The calculated DRP to IU ratio of rAAV/IgG1b12 used in these experiments was 28:1

### Example 3

### Production of Circulating IgG₁ in rAAV Transduced Mice

Immunodeficient Rag1 mice were inoculated with rAAV/IgG1b12 into both quadriceps muscles Rag1 mice were used to avoid an anti-human IgG response

All experiments were conducted in accordance with the Children's Hospital Institutional Animal Care and Use Committee. Six week old Rag-1 mice (C.129S7(B6)-Rag1*^{tm1Mom}*) were purchased from The Jackson Laboratory (Bar Harbor, Maine) and housed in microisolator barrier housing The study consisted of 16 animals: 6 received 5 x 10¹¹ DNase resistant particles (DRP) of rAAV/IgG1b12; 6 received 5 x 10¹⁰ DRP; 2 received an irrelevant rAAV vector expressing β-glucuronidase (rAAV/GUS, 4 x 10¹¹ DRP); and, 2 were given PBS diluent (used for vector DNA analysis only).

Mice were anesthetized with intramuscular injection of tiletamine HCl/zolezapam HCl (Telazol, Ft. Dodge, IA). A 5 mm skin incision was made over the distal femur and 50 µl of'the viral suspension or PBS was injected in the quadriceps femoris muscle along the long axis of the muscle using a 28-gauge needle. No adverse effects attributable to the injection procedure were noted in any mice Blood samples were collected from the retroorbital-sinus under anesthesia At the time of sacrifice, the entire quadriceps femoris muscles were removed and bisected along the transverse plane and half'fixed in a non-crosslinking fixative (Histochoice, Amresco, Solon, OH) and paraffin embedded, and the other half quick frozen in liquid nitrogen for subsequent DNA analysis. Tibialis anterior muscle was taken as control tissue.

Human IgG₁ was detected at 6 weeks post-inoculation in all eleven surviving rAAV/IgG1b12 mice; one mouse died at 2 weeks from unrelated causes On average, animals that received the higher dose of rAAV/IgG1b12 possessed 7 to 28 times more IgG₁ than lower dose animals Maximal IgG₁ concentrations were observed 12 weeks after injection, and then plateaued over the next 3 months. The majority of high dose animal sera consistently possessed between 4 and 5 µg of human IgG₁ per ml, with the maximum level exceeding 8 µg/ml. In the low dose group, antibody levels continued to increase 20 weeks after vector inoculation with circulating antibody levels in the 0 5 - 1 0 µg/ml range

Several sera were also assayed to determine an anti-HIV gp120 end-point ELISA titer The anti-HIV-1 gp120 ELISA was carried out as follows. Immulon 4 immunoassay plates (Dynatech) were coated (100 ng/well) with recombinant HIV-1_{LA1} gp120 produced in Chinese hamster ovary (CHO) cells (Quality Biological, Gaithersburg, MD) diluted in carbonate buffer (BupH, Pierce, Rockford, IL) for 16 hours at 4° C. Antigen was removed and the wells were blocked with 1% normal goat serum in Blotto (5% skim dry milk in 1x PBS pH 7.4) for 1 hour at 25° C Mouse sera were diluted in 0.1% (v/v) Triton-X100 in PBS and incubated for 30 minutes, then washed 5 times by immersion in 0.1% (v/v) Triton-X100 in PBS. A goat anti-human IgG₁ HRP conjugated secondary antibody (1:5,000) was added for 1 hr (Pierce, Rockford, IL). The colorometric substrate, 3,3',5,5' tetramethylbenzidine (TMB) was added and the reaction stopped after 30 minutes with the addition of 1 N H₂SO₄. Both ELISA assays were read at 450 nm on a Perkin-Elmer HTS 7000 plate reader. Endpoint titers were derived by taking the reciprocal of'the serum dilution that yielded OD₄₅₀ values that were at least 2 times higher than the corresponding no antigen control wells.

Serial dilutions serum taken at 16 weeks from 4 higher dose animals revealed endpoint titers in the range of 1:800 - 1:3,200. These data confirmed that muscle secreted IgG1b12 retained gp120 binding specificity.

### Example 4

### Muscle-derived IgG1b12 Neutralizes HIV-1

While the IgG₁ and gp120 ELISA data confirmed *in vivo* antibody expression, these assays did not address whether secreted IgG1b12 retained the ability to neutralize HIV-1. Therefore, 20 week serum samples from the 6 high dose animals were analyzed for neutralization activity against TCLA strain HIV-1 IIIB using the MT-2 cell-killing assays. These assays utilized Finter's neutral red to quantify viable cells as described in Herzog et al., Proc. Natl Acad. Sci. USA 94: 5804-5809, 1997. Titers were reported as the reciprocal serum dilution at which 50% of cells were protected from virus-induced killing This 50% end-point corresponds to >90% reduction in p24 Gag antigen synthesis in this assay (See Bures et al., AIDS Res. Hum. Retroviruses 16: 2019-2035, 2000). Neutralization of SHIV-89.6 was measured in mitogen-stimulated human peripheral blood mononuclear cells (PBMC) by using a reduction in p27 Gag antigen synthesis as described in Bures *et al., supra.* Virus stocks were generated in either H9 cells (HIV-1 IIIB) or human PBMC (SHIV-896).

Sera from 5 of the 6 animals possessed detectable neutralization activity as described below in Table 1

**TABLE 1. Serum neutralization titers against HIV-1 IIIB.**

| **Mouse^{a}** | **Weeks after injection (titer)^{b}** | | **Predicted IgG1b12 concentration (**µ**g/ml)^{c}** | **Observed human IgG₁ concentrarion (**µ**g/ml)^{d}** |
|---|---|---|---|---|
| | **0** | **20** | | |
| RA1 | < 20 | 93 | 6.7 | 5.1 |
| RA2 | < 20 | 56 | 4.0 | 5.9 |
| RA3 | < 20 | 55 | 4.0 | 6.0 |
| RB1 | < 20 | 44 | 3.2 | 5.3 |
| RB2 | < 20 | 47 | 3.4 | 4.0 |
| RB3 | < 20 | < 20 | 0.0 | 2.8 |

| | | | | |
|---|---|---|---|---|
| ^{a} Each mouse received a single dose of 5 x 10¹¹ DRP of rAAV/IgG1b12 in the quadriceps ^{b} Antibody-mediated neutralization was measured in an MT-2 cell-killing assay. Titers are the reciprocal serum dilution at which 50% of cells were protected from virus-induced killing, which corresponds to >90% reduction in p24 Gag antigen synthesis. ^{c} Concentration of purified IgG1b12 that is necessary to achieve the observed neutralization titer. ^{d} Actual concentration of human IgG1 in sera. | | | | |

To extend these data, the ability of mouse sera to neutralize a primary-like HIV-1 isolate (SHIV-89.6 containing the HIV 89.6 env) was also tested. Neutralization activity was observed in serum pools from 16, 20 and 24 weeks after injection (Table 2); sera were pooled because of limited volumes. These data indicated that IgG1b12 originating from muscle retained the predicted ability to neutralize both TCLA and primary HIV-1 isolates.

**TABLE 2. Serum neutralization titers against SHIV 89.6.**

| **Test sample^{a}** | | **p27 (pg/ml)** | **% reduction^{b}** |
|---|---|---|---|
| Diluent | | 1725 | 0 |
| IgG1b12 (4 µg/ml) | | 89 | 95 |
| Pooled mouse sera (week after injection) | 0 | 711 | 59 |
| | 16 | 225 | 87 |
| | 20 | 326 | 81 |
| | 24 | 527 | 69 |

| | | | |
|---|---|---|---|
| ^{a} Pre-immune (time 0) pool consisted of sera from mice RA1, RA2, RA3, RB1, RB2, and RB3. Sera from these same mice were pooled by collection date (16, 20, or 24 wks after injection) as indicated. All pools were assayed at a 1:4 dilution. ^{b} Percent reduction in p27 antigen was calculated relative to the amount of p27 synthesized in the absence of serum. | | | |

### Example 5

### rAAV Vector Persistence and Protein Production in Muscle

To obtain evidence that muscle was the site of antibody production, rAAV genome persistence and human IgG protein expression was assayed in muscle tissue harvested 24 weeks post-inoculation. rAAV/IgG1b12 vector DNA persistence was analyzed using real-time quantitative PCR. Approximately 50% of the left and right quadriceps from each animal were used for genomic DNA isolation and subjected to Taqman PCR using a PCR primer/probe pair specific for the CMV promoter. As shown in Table 3, all inoculated muscle tissue possessed significant levels of vector DNA that ranged between 0.4 - 10 copies per nucleus. On average, muscle from animals that received the higher dose possessed 5 times more vector DNA per muscle nucleus than muscle from low dose animals

**TABLE 3. Persistence of vector DNA in mouse muscle.**

| **Vector/dose^{a}** | **Mouse** | **Genome copies/nucleus^{b}** |
|---|---|---|
| **PBS** | RF3 | 0003 |
| | RF4 | 0.012 |
| **rAAV/IgG1b12 5 X 10¹⁰** | RC1 | 9.0 |
| | RC2 | 5.5 |
| | RC3 | 0.5 |
| | RD2 | 1.2 |
| | RD3 | 4.5 |
| **rAAV/IgG1b12 5 X 10¹¹** | RA1 | 42.5 |
| | RA2 | 3.2 |
| | RA3 | 20.0 |
| | RB1 | 27.1 |
| | RB2 | 0.4 |
| | RB3 | 19.3 |

| | | |
|---|---|---|
| ^{a} Dose is measured as DNase resistant particles (DRP); see Materials and Methods ^{b} Values represent the average rAAV genomes per nucleus observed in the quadriceps muscles following rAAV injection 60 ng of muscle DNA (10,000 nuclei equivalents) was analyzed by quantitative Taqman PCR using the CMV primer/probe set (SEQ ID NO: 1 AND 2). All samples were harvested 24 weeks after injection. | | |

To demonstrate *in situ* antibody expression within the inoculated muscle, immunoperoxidase staining for human IgG₁ was performed on paraffin embedded muscle tissue. Muscle tissue for human IgG₁ heavy chain (Fc specific) detection was serial sectioned (6 µm) and deparaffinized in Americlear with successive ethanol baths followed by a Ix PBS + 0.2% Tween 20 wash. Tissue sections were initially processed using the Antigen Retrieval Citra Solution (BioGenex) according to the manufacturer's instructions and then blocked for 10 minutes using Power Block reagent (BioGenex) A 1:100 dilution of a polyclonal rabbit anti-human IgG antiserum (DAKO, A0424) was incubated with the sections for 18 hr at 4° C After extensive washing, a biotinylated anti-rabbit secondary antibody (1:100 dilution; Vector Laboratories) was added and incubated for 30 minutes Antigen was visualized using an avidin/biotin - peroxidase conjugate according to the manufacturer's instructions (VECTASTAIN Elite ABC-peroxidase, Vector Laboratories). Color development was achieved by incubating the sections for 5 minutes in AEC peroxidase substrate (DAKO)

All higher dose animals demonstrated appreciable, often punctate, immunostaining of' specific myofibers consistent with ER/golgi localization of the secreted protein. Control rAAV/GUS quadriceps tissue was negative for the presence of human IgG₁ (animals RF 1, RF2)

### Example 6

### Construction of a single promoter rAAV for antibody expression

A rAAV genome comprising AAV ITRs flanking a gene cassette comprising the human CMV immediate early promoter/enhancer, the SV40 small T-antigen intron, X5 heavy chain variable region coding sequences, a (Gly₃Ser)₄ linker, X5 light chain variable region coding sequences and the SV40 polyadenylation site was constructed using standard DNA manipulation techniques. The gene cassette encoded a single chain X5 antibody polypeptide (ScFvX5). See Figure 2. The AAV ITRs were modified as described in McCarty et al., Gene Therapy, 8, 124801254, 2001 to allow for packaging of the rAAV genome into viral particles as double-stranded, self-complimentary DNA.

The ability of'the gene cassette to produce ScFvX5 was confirmed *in vitro* using HeLa cells. The gene cassette was transiently transfected into HeLa cells as naked DNA. The ScFvX5 produced by the transfected cells and was demonstrated to bind HIV-1 gp120 by standard methods in the art.

The rAAV/ScFvX5 genome was packaged essentially by the methods described in Example 2 above but was packaged into AAV serotype 1 capsid and then purified. The resulting rAAV/ScFvX5 may be tested in the mouse model described in Examples 3, 4 and 5 above and the macaque model described in Example 6 below.

### Example 7

### RAAV Administration to Macaques

Infection of macaque monkeys with SIV can induce AIDS-like disease. These infected animals can be employed as a model for infection of humans with HIV-1 and development of AIDS in humans. In particular, macaques infected with SIV virus develop manifestations characteristic of human AIDS such as depletion of CD4-positive T cells, development of opportunistic infections, neurological diseases and malignancies, and the like Moreover, SIV can infect animals by routes of administration (*e.g*., rectal and vaginal) that reproduce the transmission of HIV in humans See, for example, Nathanson, International Journal of STD & AIDS, 9(Suppl. 1):3-7 (1998). The SIV/macaque model is thus the leading animal model for AIDS vaccine development and parthenogenesis See, for example, Ho et al., Cell, 110:135-138 (2002) (discussing that the success of strategies employed in monkey experiments has "propelled a number of candidate vaccines into clinical trials") See also, grants1.nih.gov/grants/guide/rfa-files/RFA-MH-99-009 htm1 (January 29, 1999) (discussing the "importance of the SIV model of AIDS to aid in deciphering and understanding the mechanisms underlying human AIDS neuropathogenesis").

Moreover, challenge studies in monkeys can be employed to establish protection against immunodeficiency viruses and/or the diseases resulting from infection with the viruses and are tools for establishing whether a vaccine or vaccine concept holds promise for its use in humans See Schultz, AIDS Research and Human Retroviruses, 14(3):S261-S263 (1998).

The macaque model can thus be utilized to confirm the beneficial effects of' administration of rAAV/IgG1b12 and/or rAAV encoding other HIV-1 neutralizing antibody For example, rAAV/IgG1b12 is administered by one or more intramuscular injection(s) to macaques prior, or subsequent to, infection with a challenge HIV-1 virus such as SIVsm/E660 (a viral strain that induces a more AIDS-like disease in macaques presenting a more stringent challenge than other commonly-employed challenge viruses). Neutralizing antibody levels are measured at various time points as described in Polacino et al., J. Virol, 73(10): 8241-8215 (1999).

Administration of the rAAV results in one or more of the following in macaques exposed to SIV: a reduction of viral loads, maintenance of' low viral loads, an increase in CD4-positive T cells, stabilization of CD4-positive T cells, reduced incidence or severity of opportunistic infections, reduced incidence of malignancies, and reduced incidence or severity of conditions typical of defects in cell-mediated immunity. The foregoing are each in comparison to a macaque that, according to the art, has progressed or will likely progress to AIDS-like disease

### Example 8

### Administration of rAAV Encoding HIV-1 Antibody Polypeptides to Humans

rAAV/IgGlb12, rAAV/ScFvX5, rAAV encoding other HIV-1 antibody polypeptide (or a mixture of two or more of the following) is administered to an individual susceptible to infection by HIV-1 or infected by HIV-1 to prevent or slow progression to AIDS.

For example, one or more intramuscular injection(s) of rAAV is(are) administered to the individual. Levels of production of antibody polypeptide encoded by the rAAV are monitored by techniques standard in the art Likelihood of progression to AIDS is monitored by measurement, by techniques standard in the art, of HIV-1 viral loads and CD4-positive Tcells. The higher the viral loads, the more likely the individual is to develop AIDS. The lower the CD4-positive T cell count, the more likely the individual is to develop AIDS.

Administration of the rAAV results in one or more of the following in the individual when infected with HIV-1: a reduction of viral loads, maintenance of low viral loads, an increase in CD4-positive T cells, stabilization of CD4-positive T cells, reduced incidence or severity of opportunistic infections, reduced incidence of malignancies, and reduced incidence or severity of conditions typical of defects in cell-mediated immunity. The foregoing are each in comparison to an individual that, according to the art, has progressed or will likely progress to AIDS.

### Example 9

### rAAV Encoding Other Antibody Polypeptides

Other rAAV may be generated and used according to the methods described herein such as rAAV encoding antibody polypeptides Orthoclone OKT3 for allograft rejection, ReoPro as a PTCA adjunct, Rituxan for non-Hodgkin's lymphoma, Simulect for organ rejection, Remicade for rheumatoid arthritis and Crohn's disease, Zenapax for organ rejection, Synagis for RSV infection, Herceptin for metastatic breast cancer, Mylotarg for acute myeloid leukemia, Campath for chronic lymphocytic leukemia, Zevalin for non-Hodgkin's lymphoma and Humira for rheumatoid arthritis. These have been approved for human use for the infections or disease states noted.

While the present invention has been described in terms of specific embodiments, it is understood that variations and modifications will occur to those skilled in the art. Accordingly, only such limitations as appear in the claims should be placed on the invention.

The invention will now be defined by the following clauses:
1. The rAAV/IgG1b12 genome.
2. A polynucleotide comprising the rAAV/IgG1b12 genome of clause 1.
3. A packing cell comprising the rAAV/IgG1b12 genome of clause 1.
4. A purified rAAV/IgG1b12 comprising the rAAV/IgG1b12 genome of clause 1.
5. A composition comprising the rAAV/IgG1b12 of clause 4.
6. A method of producing IgG1b12 antibody in a cell comprising the step of transducing a cell with the purified rAAV/IgG1b12 of clause 4 in an amount effective to elicit expression of IgG3b12 antibody in said cell.
7. A method of producing IgG1b12 antibody in an animal comprising the step of administering to said patient the composition of clause 5 in an amount effective to elicit expression of IgG1b12 antibody in said animal.
8. The method of clause 7 wherein said animal is a human being
9. The rAAV/ScFvX5 genome.
10. A polynucleotide comprising the rAAV/ScFvX5 genome of clause 9.
11. A packaging cell comprising the rAAV/ScFvX5 genome of clause 9.
12. A purified rAAV/ScFvX5 comprising the rAAV/ScFvX5 genome of clause 9.
13. A composition comprising the rAAV/ScFvX5 of clause 12.
14. A method of producing ScFvX5 antibody polypeptide in a cell comprising the step of transducing a cell with the purified rAAV/ScFvX5 of clause 12 in an amount effective to elicit expression of ScFvX5 antibody polypeptide in said cell.
15. A method of producing ScFvX5 antibody polypeptide in an animal comprising the step of administering to said patient the composition of clause 13 in an amount effective to elicit expression of ScFvX5 antibody polypeptide in said animal.
16. The method of clause 15 wherein said animal is a human being
17. A purified rAAV encoding an anti-HIV-1 antibody polypeptide.
18. A composition comprising one or more rAAV of clause 17
19. A method of producing anti-HIV-1 antibody polypeptide in a cell comprising the step of transducing a cell with the rAAV of clause 17 in an amount effective to elicit expression that anti-HIV-1 antibody polypeptide in said cell.
20. A method of producing anti-HIV-1 antibody polypeptide in an animal comprising the step of administering to said patient the composition of clause 19 in an amount effective to elicit expression of the anti-HIV-1 antibody polypeptide in said animal.
21. The method of clause 20 wherein said animal is a human being

## Claims

1. A rAAv composition for use in a method of producing antibody polypeptides in an animal, the method comprising the step of transducing muscle, liver or brain cells of the animal with an amount of the rAAV composition effective to elicit expression of the antibody polypeptides, wherein the rAAV composition comprises rAAV encoding an antibody polypeptide or comprises a mixture of two or more rAAV encoding different antibody polypeptides.

2. The rAAV composition of claim 1 wherein the animal is a human being.

3. The rAAV composition of claim 1 or 2 wherein an antibody polypeptide is directed to a viral protein.

4. The rAAV composition of claim 3 wherein the viral protein is a human immunodeficiency virus protein.

5. The rAAV composition of claim 4 wherein the rAAV composition comprises rAAV/IgG1b12; a gene cassette comprising the human CMV intermediate early promoter/enhancer, the SV40 small T-antigen intron, b12 heavy chain coding sequences, the bovine growth hormone polyadenylation site, the human elongation factor-1α promoter modified using the R segment and part of the U5 sequence of the HTLV Type 1 Long Terminal Repeat, the I117 intron, b12 light chain sequences and the SV40 polyadenylation site.

6. The rAAV composition of claim 5 wherein the rAAV composition comprises rAAV/ScFvX5; a gene cassette comprising the human CMV intermediate early promoter/enhancer, the SV40 small T-antigen intron, X5 heavy chain variable region coding sequences, a (Gly₃ser)₄ linker, X5 light chain variable coding sequences and the SV40 polyadenylation site.

7. The rAAV composition of claim 1 or 2 wherein an antibody polypeptide is directed to a bacterial protein.

8. The rAAV composition of claim 1 or 2 wherein an antibody polypeptide is directed to a protein associated with a chronic disease state.

9. A purified rAAV encoding an anti-HIV-1 antibody polypeptide.

10. The purified rAAV of claim 9 encoding an IgG1b12 antibody.

11. The purified rAAV of claim 9encoding an ScFvX5 antibody.

12. A composition comprising one or more rAAV of claim 9.

13. A purified rAAV of claim 9 for use in a method of producing anti-HIV-1 antibody polypeptide in a cell, the method comprising the step of transducing a cell with said rAAV in an amount effective to elicit expression of the anti-HIV-1 antibody polypeptide in said cell.

14. A composition of claim 12 for use in a method of producing anti-HIV-1 antibody polypeptide in an animal, the method comprising the step of administering to said patient said composition in an amount effective to elicit expression of the anti-HIV-1 antibody polypeptide in said animal.

15. The purified rAAV of claim 14, wherein said animal is a human being.
